(19) Europäisches Patentamt
European Patent Office
Office européen des brevets

(11) **EP 2 153 210 B1**

(12) **EUROPÄISCHE PATENTSCHRIFT**

(45) Veröffentlichungstag und Bekanntmachung des Hinweises auf die Patenterteilung:
**03.11.2010 Patentblatt 2010/44**

(51) Int Cl.:
**G01N 21/65** *(2006.01)* **G01J 3/44** *(2006.01)*
**G02F 1/35** *(2006.01)*

(21) Anmeldenummer: **08758380.3**

(22) Anmeldetag: **05.05.2008**

(86) Internationale Anmeldenummer:
**PCT/EP2008/003582**

(87) Internationale Veröffentlichungsnummer:
**WO 2008/135257 (13.11.2008 Gazette 2008/46)**

(54) **VERFAHREN UND OPTISCHE ANORDNUNG ZUM ERZEUGEN EINES NICHT-LINEAREN OPTISCHEN SIGNALS AN EINEM DURCH EIN ANREGUNGSFELD ANGEREGTEN MATERIAL SOWIE VERWENDUNG DES VERFAHRENS UND DER OPTISCHEN ANORDNUNG**

METHOD AND OPTICAL ARRANGEMENT FOR GENERATING A NONLINEAR OPTICAL SIGNAL ON A MATERIAL WHICH IS EXCITED BY AN EXCITATION FIELD, AND USE OF THE METHOD AND OF THE OPTICAL ARRANGEMENT

PROCÉDÉ ET DISPOSITIF OPTIQUE POUR LA PRODUCTION D'UN SIGNAL OPTIQUE NON LINÉAIRE SUR UN MATÉRIAU EXCITÉ PAR UN CHAMP D'EXCITATION, ET UTILISATION DU PROCÉDÉ ET DU DISPOSITIF OPTIQUE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **04.05.2007 DE 102007021378**

(43) Veröffentlichungstag der Anmeldung:
**17.02.2010 Patentblatt 2010/07**

(73) Patentinhaber: **APE Angewandte Physik Und Elektronik Gmbh**
**13053 Berlin (DE)**

(72) Erfinder:
• **RIMKE, Ingo**
**10999 Berlin (DE)**
• **OTTO, Cees**
**7559 ND Hengelo (NL)**
• **BÜTTNER, Edlef**
**12623 Berlin (DE)**
• **OFFERHAUS, Hermann, L.**
**7415 CH Deventer (NL)**
• **JURNA, Martin**
**7558 BN Hengelo (NL)**

(74) Vertreter: **Eisenführ, Speiser & Partner**
**Anna-Louisa-Karsch-Strasse 2**
**10178 Berlin (DE)**

(56) Entgegenhaltungen:
• **BUTTNER EDLEF ET AL: "CARS imaging with a new, 532 nm synchronously pumped picosecond OPO" PROGR. BIOMED. OPT. IMAGING PROC. SPIE; PROGRESS IN BIOMEDICAL OPTICS AND IMAGING - PROCEEDINGS OF SPIE; MULTIPHOTON MICROSCOPY IN THE BIOMEDICAL SCIENCES VII 2007, Bd. 6442, 10. Februar 2007 (2007-02-10), XP002489933 in der Anmeldung erwähnt**
• **JURNA M ET AL: "Noncritical phase-matched lithium triborate optical parametric oscillator for high resolution coherent anti-Stokes Raman scattering spectroscopy and microscopy" APPLIED PHYSICS LETTERS, AIP, AMERICAN INSTITUTE OF PHYSICS, MELVILLE, NY, Bd. 89, Nr. 25, 21. Dezember 2006 (2006-12-21), Seiten 251116-251116, XP012087711 ISSN: 0003-6951**
• **POTMA E O ET AL: "High repetition rate femtosecond lightsource for CARS microscopy" CONFERENCE DIGEST. 2000 CONFERENCE ON LASERS AND ELECTRO-OPTICS EUROPE (CAT. NO.00TH8505) IEEE PISCATAWAY, NJ, USA, 2000, Seite 1 pp., XP002489934 ISBN: 0-7803-6319-1**

- GREVE M ET AL: "Gated heterodyne coherent anti-Stokes Raman scattering for high-contrast vibrational imaging" PROCEEDINGS OF THE SPIE - THE INTERNATIONAL SOCIETY FOR OPTICAL ENGINEERING SPIE-INT. SOC. OPT. ENG USA, Bd. 5856, Nr. 1, 2005, Seiten 41-48, XP002489935 ISSN: 0277-786X

- GREVE M ET AL: "High-contrast chemical imaging with gated heterodyne coherent anti-Stokes Raman scattering microscopy" APPLIED PHYSICS B (LASERS AND OPTICS) SPRINGER-VERLAG GERMANY, Bd. B81, Nr. 7, November 2005 (2005-11), Seiten 875-879, XP002489936 ISSN: 0946-2171

**Beschreibung**

Die Erfindung betrifft ein Verfahren zum Erzeugen eines nicht-linearen optischen Signal gemäß Anspruch 1. Die Erfindung betrifft auch eine Verwendung des Verfahrens und der optischen Anordnung.

Die Verwendung eines vorgenannten CARS-Verfahrens und/oder CARS-optischen Anordnung hat sich insbesondere im Bereich der Untersuchung interner vibronischer Strukturen von Molekülen etabliert, wobei beispielsweise ein nicht-lineares optisches Signal in Form eines CARS-Signals (Coherent Anti-Stokes Raman Spectroscopy Signal) an einem Material erzeugt werden kann. Beispielsweise ist im Artikel von F. Ganikhanov et.al, in: Optics Letters, Vol. 31 (12), Seiten 1872 - 1874 (2006) ein hochsensitiver CARS-Aufbau beschrieben, der mit zwei Lasern und einem resonator-intern frequenz-verdoppelten OPO arbeitet. Dies ermöglicht eine markerfreie Identifizierung eines Moleküls. So erweisen sich beispielsweise Fluoreszenz-Spektroskopie gemarkerte Spezies als mögliche Alternative aber insbesondere im biologischen oder im Nahrungsmittelbereich als nicht anwendungsbezogen aufgrund der oftmals zu geringen Markerwirkung und ggf. toxischen Wirkung der Marker. Das eingangs genannte Verfahren und die Vorrichtung bilden in diesem Bereich somit eine ausbaufähige und gangbare Möglichkeit, wenn es gelingt die Komplexität bestehender Systeme zu verringern und gleichzeitig deren Sensitivität zu erhöhen. Die dem CARS-Prozess inhärente resonante Überhöhung macht diesen sensitiver als Raman-Spektroskopie-Methoden und im Vergleich zur Infrarot-Spektroskopie ermöglicht die Verwendung kürzerer Wellenlängen im vorliegenden Fall eine höhere räumliche Auflösung im Bereich der Mikroskopie. U.a. deswegen hat die Verwendung eines eingangs genannten Verfahrens und der eingangs genannten Vorrichtung im Rahmen der CARS-Spektroskopie und Mikroskopie eine nicht unbeachtliche Bedeutung erlangt. Die Implementierung derselben in größerem, anwendungsbezogenem oder industriellem Rahmen ist bislang begrenzt aufgrund der immer noch vergleichsweisen hohen Komplexität der dafür notwendigen Systeme und der vergleichsweise geringen Ausbeute des nicht-linearen optischen Signals, welches mittels einer zur Verfügung stehenden hochgradig nicht-linearen Wechselwirkungen im Material erzeugt wird. Diese Problematik besteht insbesondere bei Materialen, in denen die zu untersuchende Substanz in vergleichsweise niedriger Konzentration vorliegt.

So ist durch die Veröffentlichung von E.O. Potma et.al. in Opt.Lett. 31 Nr. 2, Seite 241 bis 243, 14. Januar 2006 "Heterodyne coherent anti-Stokes Raman scattering (CARS) imaging" und in der Veröffentlichung von E.R. Andresen in Opt. Expr. Vol. 14(16), Seiten 7246-7251 (2006) "Picosecond anti-Stokes generation in a photonic-crystal fiber for interferometric CARS microscopy" grundsätzlich vorgeschlagen worden, eine eingangs genannte Multistrahlanordnung für ein Verfahren bzw. einen optischen Aufbau zum Erzeugen des nicht-linearen optischen Signals durch die Verwendung eines optischen parametrischen Oszillators und eines heterodynen Detektionsschemas weiter zu bilden. Dazu sieht E.O. Potma et.al. vor, für eine CARS-Anordnung einen im Resonator des optischen parametrischen Oszillators (OPO) frequenz-verdoppelten Puls eines Signal-Strahls als hochfrequentes Anregungsfeld und einen von einem Pumplaser für den OPO erzeugten Laserpuls als niederfrequentes Anregungsfeld - in der CARS-Terminologie als Stokes-Strahl - zu verwenden. Zur Realisierung eines heterodynen Detektionsschematas wird in definierter Weise im CARS-Aufbau ein zusätzlicher CARS-Aufbau im Rahmen eines Mach-Zehnder-Interferometers zur Verfügung gestellt, wobei einer der Arme des Interferometers eine Zelle von deuteriertem Dimethyl-Sulfoxid (d-DMSO) zum Erzeugen eines starken nichtresonanten Signals bei einer - in der CARS-Terminologie Anti-Stokes-Frequenz - zu erzeugen, welches als sogenannter "lokaler Oszillator" im Rahmen des heterodynen Detektionsschemas dient. Ein solcher Aufbau ist vergleichsweise komplex und aufwändig zu handhaben, obgleich das vorgeschlagene heterodyne Detektionsschema zur Erhöhung einer Sensivität des eingangs genannten Verfahrens und der eingangs genannten Vorrichtung grundsätzlich zweckmäßig ist.

In der Veröffentlichung von E.R. Andresen et al. dient wiederum ein Signalausgang eines auf einer Laserfrequenz synchron gepumpten OPO's als hochfrequenter Pumppuls für das Anregungsfeld zur Erzeugung eines CARS-Signals, während ein Laserpuls auf der Laserfrequenz als niederfrequenter Stokes-Puls zur Bildung des Anregungsfeldes dient. Pump- und Stokes-Puls werden zur Darstellung eines lokalen Oszillatorfeldes im Anti-Stokes-Puls im Rahmen einer heterodynen Detektionsschematas für CARS einer photonischen kristallinen Glasfaser (photonic-crystal fiber - PCF) zugeführt. Die damit verbundenen Dispersions- und Laufzeiteigenschaften betreffend den Anti-Stokes-Puls erzeugen zusätzliche Probleme, welche das dort beschriebene Verfahren und den dort beschriebenen Aufbau noch verbesserungswürdig machen.

In der Veröffentlichung "CARS imaging with a new 532 nm synchroneously pumped picosecond OPO" von Büttner et.al, erschienen in Proc. of SPIE Conf. Multiphoton Microscopy in the Biomedical Sciences VII (21.-23. Jan. 2007), edt. by A. Periasamy u.a., SPIE, 2007 Vol. 6442, S 64420C-1 bis 64420C-8, ist grundsätzlich ein vorteilhafter optischer CARS-Aufbau mit einem OPO beschrieben, der mit einem frequenzverdoppelten modengekoppelten Nd:VAN-Laser gepumpt wird. Dort wird ein Signal-Strahl des OPO's zusammen mit einem Teil der Fundamentalen des Pump-Lasers als Pump- und Stokes-Strahl am Rahmen eines CARS-Anregungsfeldes genutzt.

Wünschenswert wäre ein vergleichsweise leistungsstarker und kompakter Ansatz zur weiteren Verbesserung eines eingangs genannten Verfahrens und einer eingangs genannten optischen Anordnung, wobei darüber hinaus eine vorteilhafte Möglichkeit einer heterodynen Detektion für das nicht-lineare optische Signal bestehen sollte.

An dieser Stelle setzt die Erfindung an, deren Aufgabe es ist, ein Verfahren und eine optische Anordnung der eingangs genannten Art anzugeben, welche vergleichsweise einfach und/oder kompakt im Aufbau und dabei dennoch vergleichsweise leistungsstark zum Erzeugen des nicht-linearen optischen Signals, insbesondere vergleichsweise sensitiv zur Detektion des nicht-linearen optischen Signals, genutzt werden können.

Betreffend das Verfahren wird die Aufgabe durch die Erfindung mittels einem Verfahren der eingangs genannten Art gelöst, bei dem erfindungsgemäß vorgesehen ist, dass die zweite optische Generatoreinheit als optische parametrische Generatoreinheit Pulse einer Idler-Frequenz in einem Idler-Strahl zur Bereitstellung der zweiten Pulse der zweiten Frequenz in dem zweiten Strahl erzeugt, wobei die zweite Frequenz kleiner als die erste Frequenz ist und die zweite optische Generatoreinheit Pulse einer Signalfrequenz in einem Signalstrahl erzeugt, wobei die zweite Frequenz kleiner als die Signal-Frequenz ist.

Die Erfindung geht von der Überlegung aus, dass es in Abgrenzung zum Stand der Technik, möglich ist eine besonders leistungsstarke Anregung eines Materials im Rahmen eines Anregungsfeldes zu erreichen, welches mittels eines eines ersten Pulses, z.B. von einem Pumplaser erzeugten Pumppulses höherer Frequenz, und eines zweiten Pulses, z.B. von einem optischen parametrischen Oszillator erzeugten Idler-Pulses niedrigerer Frequenz, gebildet wird. Mit anderen Worten, die fundamentale Frequenz des, vorzugsweise Lasers, dient als Pumpfrequenz (erster Puls) im Rahmen einer CARS-Terminologie, während die Idler-Frequenz, vorzugsweise des OPO's, als Stokes-Frequenz (zweiter Puls) dient. Die Erfindung hat erkannt, dass diese Art der Erzeugung eines Anregungsfeldes mit dem zweiten Puls möglich ist, wenn gemäß dem Konzept der Erfindung die optische parametrische Generatoreinheit auf der durch Vervielfachung der fundamentalen Frequenz gebildeten höheren harmonischen Frequenz gepumpt wird.

Darüber hinaus hat die Erfindung erkannt, dass die Kombination der ersten, fundamentalen Frequenz und der zweiten Idler-Frequenz im Anregungsfeld zu einem nicht-linearen Signal, z.B. einem CARS-Signal, auf der Frequenz

$$\Omega_{CARS} = 2\Omega_{pump} - \Omega_{Idler}$$

führt, welches der Signalfrequenz $\Omega_{signal}$ des z.B. vom parametrischen Oszillator erzeugten Signalpulses in einem Signalstrahl entspricht. Damit bietet das vorliegende Verfahren zum Erzeugen des nicht-linearen optischen Signals erstmals eine besonders gute und leistungsstarke Möglichkeit den von der optischen parametrischen Generatoreinheit erzeugten Signalpuls im Rahmen eines heterodynen Detektionsschemas für das nicht-lineare optische Signal zu verwenden. Das Verfahren und eine dazu geeignete optische Anordnung erweisen sich als vergleichsweise kompakt und einfach handhabbar im Vergleich zu bisherigen Konzepten.

Dementsprechend führt das Konzept der Erfindung auf eine optische Anordnung der eingangs genannten Art, welche sich insbesondere zur Durchführung des Verfahrens gemäß dem Konzept der Erfindung eignet. Die optische Anordnung weist erfindungsgemäß weiter auf:

eine erste optische Generatoreinheit zum Erzeugen der ersten Pulse einer ersten Frequenz in einem ersten Strahl, ein Mittel zum Erzeugen von Pulsen einer höheren harmonischen Frequenz mit den Pulsen der ersten Frequenz als fundamentale Frequenz, eine von der ersten optischen Generatoreinheit synchron mit den Pulsen der höheren harmonischen Frequenz pumpbare, zweite optische Generatoreinheit in Form einer optischen parametrischen Generatoreinheit zum Erzeugen von Pulsen einer Idler-Frequenz in einem Idler-Strahl als zweite Pulse in dem zweiten Strahl auf einer zweiten Frequenz und zum Erzeugen von Pulsen einer Signal-Frequenz in einem Signalstrahl, wobei die zweite Frequenz kleiner als die erste Frequenz und kleiner als die Signal-Frequenz ist, Strahlführungsmittel zum zeitlichen und örtlichen Überlappen eines ersten Pulses des ersten Strahls und eines zweiten Pulses des Idler-Strahls.

Unter einer optischen parametrischen Generatoreinheit ist grundsätzlich jede optische Generatoreinheit zu verstehen, die ausgelegt ist, über einen parametrischen Prozess einer Frequenzaufspaltung der Pumpfrequenz eine Idler-Frequenz und eine Signal-Frequenz zu erzeugen, wobei die Summe der Idler-Frequenz und Signal-Frequenz die Pumpfrequenz ergibt. Grundsätzlich kann anstatt eines OPO's auch ein optischer parametrischer Generator (OPG) - also ein OPO ohne Resonator - oder ein optischer parametrischer Verstärker mit oder ohne Resonator (OPA) verwendet werden. Die Generatoreinheiten sind vorzugsweise als Pikosekunden (ps)-Version ausgeführt, gegebenenfalls auch als Femtosekunden (fs)-Version. Ein OPO oder OPG oder OPA nutzt bevorzugt einen KTP-Kristall als nicht-linearen Kristall. Die Verwendung von ps-Pulsen erlaubt spektral gezielte Untersuchungen spezieller Übergange (Single-line) während fs-Pulse frequentiell breite Anwendungen z.B. mit Frequenzmodulation o.ä. erlauben (multi-line).

Das Konzept der Erfindung nutzt in vorteilhafter Weise, dass die Summe aus Idler-Frequenz und Signal-Frequenz der höheren harmonischen Frequenz, insbesondere der zweiten harmonischen Frequenz, entspricht. Dies ermöglicht die Option einer besonders rauschfreien Detektion des nicht-linearen optischen Signals im Rahmen eines heterodynen Detektionsschematas.

Das Konzept der Erfindung führt auf eine besonders bevorzugte Verwendung des Verfahrens und/oder der optischen Anordnung zur chemisch selektiven Untersuchung chemischer Substanzen und/oder biologischer Substanzen, insbesondere im Nahrungsmittelbereich. Die Verwendung kommt ohne invasive Marker od.dgl. aus und bietet zudem die Möglichkeit einer erhöhten Sensivität im Vergleich zum Stand der Technik. Weiter führt die Erfindung auf die Verwendung des Verfahrens oder der optischen Anordnung gemäß dem Konzept der Erfindung zur Untersuchung vibronischer Zustände in einem Molekül. Es hat sich gezeigt, dass im Rahmen der Verwendung eine Untersuchung von Substanzen auch mit einer sub-milli-molaren Konzentration eines Moleküls möglich ist.

Erfindungsgemäß wird das Verfahren und/oder die optische Anordnung gemäß dem Konzept der Erfindung zur Mikroskopie und/oder Spektroskopie, insbesondere CARS-Mikroskopie und/oder CARS-Spektroskopie eingesetzt. Erfinungsgemäß ist eine Verwendung in der Frequenzmetrologie vorgesehen, insbesondere mit phasenstabilisierten optischen Generatoreinheiten.

Die Erfindung hat erstmals erkannt, dass die Kombination der fundamentalen Frequenz der ersten optischen Generatoreiheit (z. B. Laser) und der Idler-Frequenz der zweiten optischen Generatoreinheit (z. B. OPO) zur Erzeugung des nicht-linearen Signals besonders attraktiv ist, weil dadurch der Abstand zwischen der Anti-Stokes-Frequenz und der Pump-Frequenz vergleichsweise groß ist; mit anderen Worten, die Anti-Stokes-Region ist komplett frei von einem etwaigen Frequenz-Überlapp der anregenden Pulse. Dies ist nicht der Fall für Anregungsschemata, welche die Signal-Frequenz der zweiten optischen Generatoreinheit als Pump-Frequenz und die fundamentale Frequenz der ersten optischen Generatoreinheit als Anti-Stokes-Frequenz verwenden - dort ist ein Überlapp mit dem Signal-Bereich zu erwarten im Zuge des Abtastens eines CARS-Spektrums, was den Einsatz von ggf. Probleme erzeugenden abgrenzenden Filtern erforderlich macht. Diese Maßnahme wird gemäß dem Konzept der Erfindung vermieden. Vielmehr lassen sich breitere CARS-Spektren (z.B. deutlich mehr als 80 Wellenzahlen, z.B. bei mehreren Raman-Resonanzen oder Hyperspektren auch im Bereich der Temperatur-Bandbreite) erfassen.

Neben den zuvor genannten Vorteilen hat sich darüber hinaus gezeigt, dass Dispersionsprobleme und zeitliche Jitter-Probleme sowie Pulsführungsprobleme wie bei den vergleichsweise komplexen Aufbauten des Standes Technik durch das Konzept der Erfindung vorteilhaft vermieden werden. Auch die erreichbaren Pulsbreiten - zeitlich und im Frequenzraum - lassen sich verlässlich und in überlegener Weise optimieren. Die von einer einzigen Mutter-Quelle - die erste optische Generatoreinheit - stammende kaskadierte "Farben"-Generierung im Rahmen der Erfindung macht deren Konzept besonders einfach, kompakt und anwenderfreundlich im Vergleich zu allen bekannten Systemen. Das Konzept der Erfindung bietet die Möglichkeit für eine besonders effektive und rauschfreie heterodyne Detektion, welche in noch zu erläuternder Weise die Detektion von signalschwachen nicht-linearen optischen Signalen erlauben.

Vorteilhafte Weiterbildungen sind den Unteransprüchen zu entnehmen und geben im einzelnen vorteilhafte Möglichkeiten an, das oben erläuterte Konzept im Rahmen der Aufgabenstellung sowie hinsichtlich weiterer Vorteile zu realisieren.

Es wurde erkannt, dass eine optische Phasenlage zwischen einem Puls des Signalstrahls und dem nicht-linearen optischen Signals definiert ist. Zwar ist eine Phasenlage zwischen einem Puls des ersten Strahls und einem Puls des zweiten Strahls nicht in direkter Relation zueinander, d.h. die Phasenlagen der Pulse zur Darstellung des Anregungsfeldes haben keine direkte Phasenbeziehung zueinander, jedoch ist die ausgehende Phase des nicht-linearen optischen Signals phasengelockt zur Phase des Pulses der Signalfrequenz im Signalstrahl, welcher vom optischen parametrischen Oszillator erzeugt wird. D.h., die Phase einander zugeordneter Pulse eines Signalstrahls und eines Idler-Strahls für sich sind frei. Ihre Summe ist jedoch phasengelockt zur Phase des Pumplasers. Dieser Umstand einer Phasenerhaltung erlaubt im Rahmen der Erfindung die heterodyne Detektion des nicht- linearen optischen Signals, indem dieses mit dem Signalstrahl interferierend überlappt wird. Insgesamt kann dieser Umstand dazu genutzt werden, dass ein nicht-lineares Signal interferometrisch verstärkt wird.

Vorzugsweise stimmen die Signalfrequenz und die Frequenz des nicht-linearen optischen Signals größenordnungsmäßig überein - gemäß einer Weiterbildung wird zur heterodynen Detektion des nicht-linearen Signals eine Frequenz und/oder Phase des nicht-linearen optischen Signals relativ zur Signalfrequenz und/oder Phase verändert und das nicht-lineare optische Signal als zeitabhängiges interferierendes Signal detektiert.

Vorzugsweise wird eine Frequenz und/oder Phase des nicht-linearen optischen Signals relativ zur Signalfrequenz dadurch verändert, dass die erste Frequenz und/oder Phase geändert wird. Dies hat den Vorteil, dass die erste Frequenz und/oder Phase - nämlich die fundamentale Frequenz und/oder Phase des ersten optischen Oszillators, beispielweise eines Lasers, geändert wird, welche im Rahmen des Verfahrens, beispielsweise zur Anpassung einer Frequenz des Anregungsfeldes auf ein bestimmtes Material, nicht mehr notwendigerweise durchgestimmt wird. D.h., hier liegen vergleichsweise stabile Bedingungen zur definierten Frequenz- und/oder Phasen-Änderung betreffend die heterodyne Detektion vor. Vorzugsweise wird der erste Strahl akusto-optisch moduliert, was in zweckmäßiger Weise zu einer angebrachten Frequenz- und/oder Phasen-Änderung führt - grundsätzlich ist dazu jede Art eines optischen Frequenz- und/oder Phasen-Modulators geeignet.

Insbesondere wird das nicht-lineare optische Signal in Form eines CARS-Signal also bei einer Frequenz gebildet, welche der doppelten der ersten Frequenz minus der Idler-Frequenz entspricht. Damit ist es gemäß der vorliegenden Weiterbildung erstmals möglich, im Rahmen einer Multistrahlanordnung unter Implementierung eines optischen parametrischen Oszillators zur heterodynen Detektion eines CARS-Signals jede der von den Generatoreinheiten erzeugten Frequenzen für die CARS-Frequenzen (Pump, Stokes, Anti-Stokes) zu nutzen, wobei erstmals die Phasenbeziehung des Signals des optischen parametrischen Oszillators relativ zum nicht-linearen optischen Signal ausgenutzt wird. Ermöglicht wird dies gemäß dem Konzept der Erfindung durch Pumpen der zweiten parametrischen optischen Generatoreinheit auf einer höheren harmonischen ersten optischen Generatoreinheit, wie oben erläutert.

Im Rahmen einer besonders bevorzugten Weiterbildung wird der erste und zweite Strahl kollinear überlappt um das Anregungsfeld zu bilden. Vorzugsweise wird der Signalstrahl im Rahmen einer ersten Variante nicht auf das Material geführt. Insbesondere wird der Signalstrahl zur heterodynen Detektion mit dem nicht-linearen optischen Signal kollinear überlappt, beispielsweise in einem geeigneten Detektor. Eine hochlineare Anordnung ist vergleichsweise einfach zu erreichen, grundsätzlich eignet sich jedoch jede Art eines zur Detektion ausreichenden Überlapps.

Im Rahmen einer zweiten Variante kann der Signalstrahl auch mit dem ersten und zweiten Strahl kollinear im Material überlappt werden. Im Rahmen einer ersten Abwandlung kann dazu die Intensität des Signalstrahls derart gering gewählt werden, dass eine Stärke des nicht-linearen optischen Signals unbeeinflusst vom Signalstrahl ist. Im Rahmen einer zweiten Abwandlung kann die Intensität des Signalstrahls derart hoch gewählt werden, dass eine Stärke des nicht-linearen optischen Signals mit der Intensität des Signalstrahls ansteigt. Gemäß der Weiterbildung kann also der Signalstrahl als Teil des Anregungsfeldes genutzt werden, um die Erzeugung des nicht-linearen optischen Signals zu stimulieren. Sowohl bei dieser Variante als auch bei der zuvor genannten Variante kann ein resonanter und ein nicht-resonanter Signalanteil des nicht-linearen Signals im Rahmen der heterodynen Detektion durch entsprechende Analyse des Signals separiert werden. Das stimulierte nicht-lineare optische Signal kann sehr viel höhere Pegel erreichen als die Amplitude eines bloßen Differenzsignals - letzteres im Falle eines Signalstrahls vor dem Material mit geringerer Intensität bzw. eines Signalstrahls hinter dem Material. Die intensitätsstarke Anwendung auch des Signalstrahls im Material ändert gemäß der Erkenntnis dieser Weiterbildung in stimulierender Weise die nicht-lineare Wechselwirkung im Material derart, dass z.B. einer der wechselwirkenden Übergange einer bestimmten Anregung getrieben wird. Vorzugsweise werden Pump-, Idler- und Signalstrahl mit praktisch gleicher Intensität auf das Material gegeben. Diese Art der "stimulierten" CARS-Anwendung, bei der das CARS-Feld stimulierend "gesät" wird, bietet eine überraschend effektive und völlig neuartige Möglichkeit der Spektroskopie und/oder Mikroskopie.

Des weiteren hat es sich als vorteilhaft erwiesen, je nach Bedarf, gewisse Polarisationsanordnungen für ein Anregungsfeld zu wählen, um bestimmte Materialeigenschaften untersuchen zu können. Während ansonsten die Felder des ersten und zweiten Strahls kopolarisiert sein können, bietet eine nicht-kopolarisierte, z.B. kreuzpolarisierte, Anordnung den Vorteil, dass ein nicht-resonanter Teil eines nicht-linearen Signals unterdrückt werden kann, so dass nur die resonanten Anteile interferieren und detektiert werden. Des weiteren kann die Polarisation der Felder des ersten und zweiten Strahls so gewählt werden, dass das Anregungsfeld zirkular oder elliptisch polarisiert ist. Dieses hat sich insbesondere zur Untersuchung einer Doppelbrechung oder einer Händigkeit/Chiralität von Molekülen erwiesen, wobei ein entsprechender Molekülzustand durch eine angepasste Polarisation des Anregungsfeldes abgeprüft werden kann.

Vorteilhafte Weiterbildungen der Erfindung hinsichtlich der optischen Anordnung sind den Unteransprüchen zu entnehmen und geben im Einzelnen vorteilhafte Möglichkeiten an, das oben erläuterte Konzept im Rahmen der Aufgabenstellung sowie hinsichtlich weiterer Vorteile zu realisieren. So hat sich die Weiterbildung der Vorrichtung als besonders vorteilhaft erwiesen zum Detektieren des nicht-linearen optischen Signals durch heterodynes interferierendes Überlappen mit einem weiteren Strahl. Vorzugsweise ist ein Mittel zur Veränderung der Frequenz des nicht-linearen optischen Signals relativ zur Signal-Frequenz des Signalstrahls vorgesehen und ein Detektor und weitere Strahlführungsmittel zum heterodynen interferierenden Überlappen des nicht-linearen optischen Signals mit dem Signalstrahl in dem Detektor.

Der erste optischen Oszillator ist vorzugsweise in Form eines Lasers gebildet, beispielsweise ein Nd: YAG-Laser oder Titan-Saphir-Laser.

Im Rahmen einer besonders bevorzugten Weiterbildung sieht die Vorrichtung Mittel zur Erzeugung einer höheren, harmonischen Frequenz in Form eines Frequenzverdopplers vor. Insbesondere kann dies durch die Anordnung einer oder mehrer nicht-linearer Kristalle in einem geeigneten mit Verstellmechanismen versehenen Gehäuse und/oder einem zusätzlichen Resonator realisiert sein.

Im Rahmen einer Variante kann zusätzlich oder alternativ das Mittel zur Erzeugung einer höheren, harmonischen Frequenz zwischen dem ersten optischen Oszillator und dem zweiten optischen Oszillator angeordnet sein bzw. im Rahmen einer anderen Variante, zusätzlich oder alternativ das Mittel zur Erzeugung einer höheren, harmonischen Frequenz im Resonator des ersten optischen Oszillators gebildet sein. Beispielsweise kann ein Laser zur Verfügung gestellt werden, der im Rahmen einer im Laserresonater durchgeführten Frequenzverdopplung sowohl die erste Frequenz als fundamentale Frequenz als auch eine höhere, harmonische der fundamentalen Frequenz, insbesondere die doppelte harmonische der fundamentalen Frequenz erzeugt.

Grundsätzlich kann ein Mittel zur Veränderung der Frequenz und/oder der Phase des nicht-linearen optischen Signals relativ zur Frequenz und/oder der Phase des Signalstrahls zusätzlich oder alternativ zum Mittel zur Veränderung der fundamentalen Frequenz/Phase im ersten Strahl auch zur Veränderung der Idler-Frequenz/Phase im Idler-Strahl und/oder zur Veränderung der Signalfrequenz/Phase im Signalstrahl angeordnet sein. Dies kann beispielsweise ein akusto-optischer Modulator sein - grundsätzlich ist jede geeignete Wahl eines Frequenz- und/oder Phasenmodulators möglich.

Ein Detektor ist vorzugsweise in Form eines Photomultipliers, einer Photodiode oder einer Avalanche-Diode gebildet. Es hat sich überraschenderweise gezeigt, dass das Rauschverhalten, insbesondere für den Bereich einer heterodynen Detektion, im Falle einer Photodiode besonders vorteilhaft ist. Zwar ist die Verwendung eines Photomultipliers grundsätzlich bei niedrigen Signalpegeln zu empfehlen, doch hat sich selbst für niedrige Signalpegel, insbesondere gemäß der oben erläuterten Weiterbildung eines stimulierten Generierens des nicht-linearen Signals, eine Möglichkeit ergeben, den Signalpegel des nicht-linearen Signal anzuheben und dadurch die Verwendung einer rauschärmeren Photodiode, insbesondere mit einem Widerstand oberhalb von 1MΩ , bevorzugt bis zu oder oberhalb von 10 MΩ, zu ermöglichen. Damit wird erstmals ein Schrotrauschen begrenztes heterodynes Detektieren eines nicht-linearen Signals, insbesondere eines CARS-Signals, möglich.

Ausführungsbeispiele der Erfindung werden nun nachfolgend anhand der Zeichnung im Vergleich zum Stand der Technik, welcher zum Teil ebenfalls dargestellt ist, beschrieben. Diese soll die Ausführungsbeispiele nicht notwendigerweise maßstäblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in der Zeichnung sowie in den Ansprüchen offenbarten Merkmale der Erfindung können sowohl einzeln als auch in beliebiger Kombination für die Weiterbildung der Erfindung wesentlich sein. Die allgemeine Idee der Erfindung ist nicht beschränkt auf die exakte Form oder das Detail der im folgenden gezeigten und beschriebenen bevorzugten Ausführungsform oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein.

Im Einzelnen zeigt die Zeichnung in:

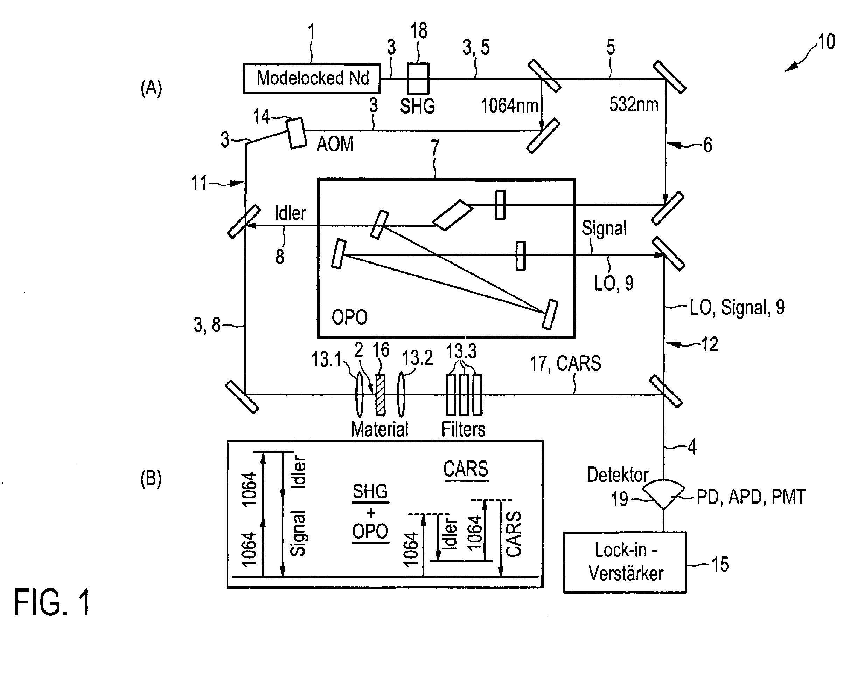

Fig. 1: in Ansicht A eine schematische Darstellung eines optischen Aufbaus zur Erzeugung und heterodynen Detektion eines CARS-Signals mit einem auf der zweiten Harmonischen, einer fundamentalen Laserfrequenz synchron gepumpten optischen parametrischen Oszillator, wobei die fundamentale Laserfrequenz und die Idler-Frequenz zur Erzeugung eines Anregungssignals für ein Material genutzt und die Signalfrequenz zur heterodynen Detektion des CARS-Signals genutzt wird; in Ansicht B ein zugehöriges Energieschemata der beteiligten Frequenzen und das Zustandekommen des CARS-Signals im Energieschema für einen nicht- linearen Prozess dritter Ordnung;

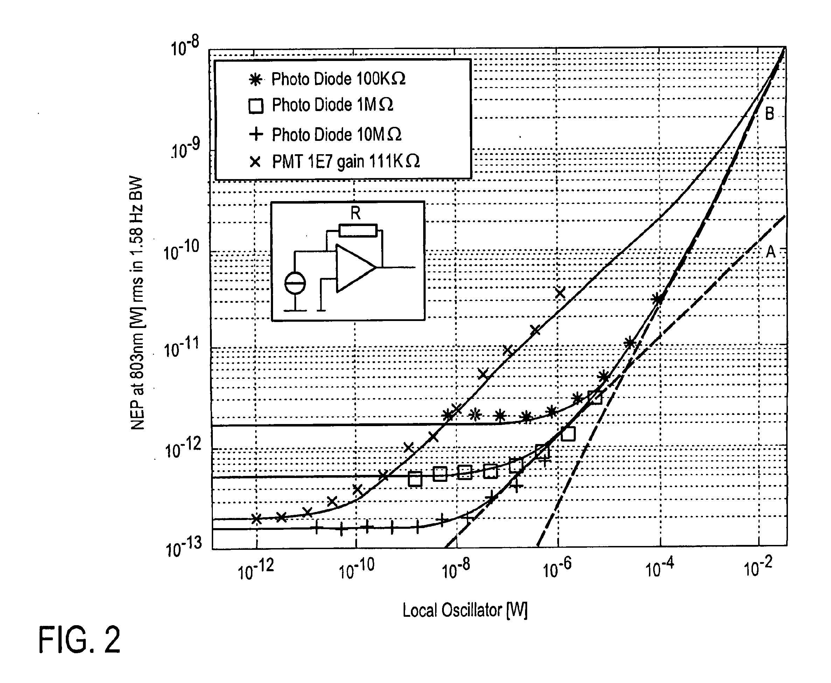

Fig. 2: das Rauschen für unterschiedliche Detektoren als Funktion einer lokalen Oszillatorstärke, d.h. des Signal-Strahls bzw. Signal-Pulses, wobei die gestrichelten Linien A und B jeweils eine Steigung von ½ bzw. 1 haben und die kontinuierlichen Linien eine Simulation darstellen, wobei die Symbole gemessene Daten repräsentieren - der Einschub zeigt eine zugehörige Detektorelektronik;

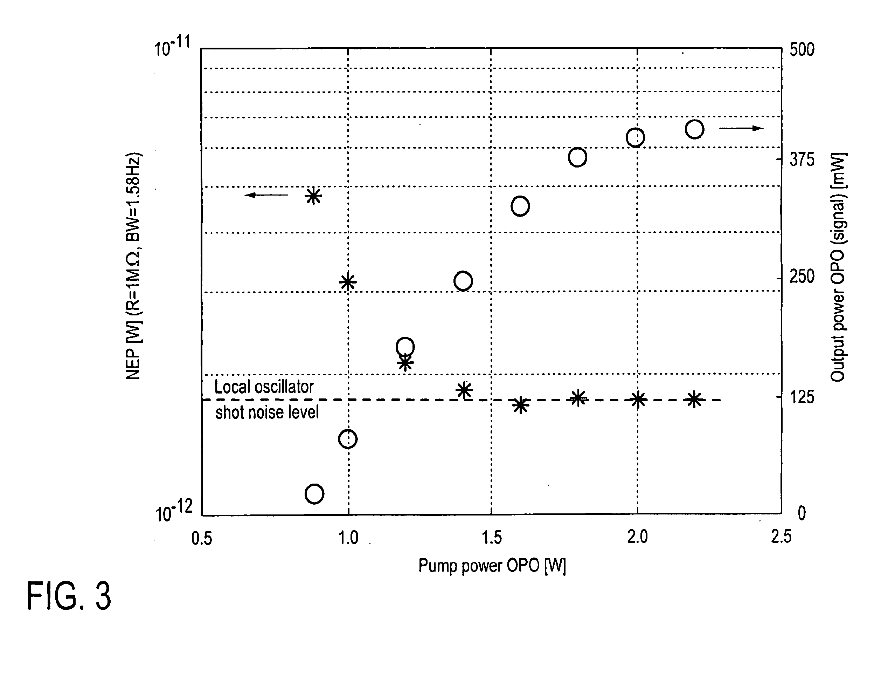

Fig. 3: das Rauschen für ein nicht-lineares optisches Signal mit 2 Mikrowatt Leistung und der gesamten Signalausbeute aufgetragen über eine Pumpleistung für den optischen parametrischen Oszillator (bei 532 nm innerhalb des Kristalls);

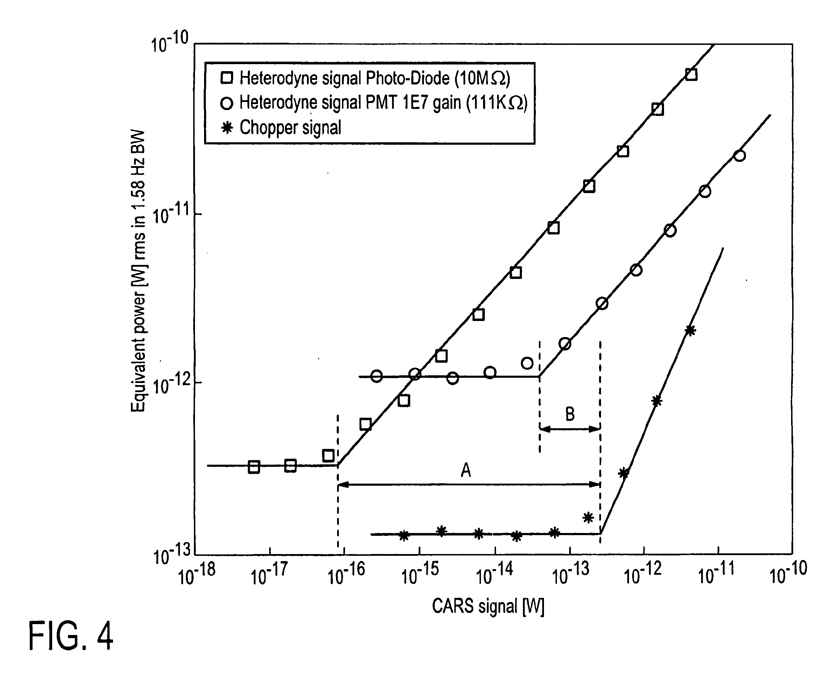

Fig. 4: eine Grafik zur Gegenüberstellung eines heterodyn detektierten nicht-linearen optischen Signals gegenüber einem direkt detektierten Signal aufgetragen über eine CARS-Leistung; Ansicht (A) repräsentiert einen Verbesserungsfaktor (vorliegend 3500) einer Detektionssensivität zwischen einer heterodynen Detektion und einer direkten Detektion (beispielsweise mit einen Chopper und einer Lock-In-Technik) eines nicht- linearen Signals auf einer Photodiode; Ansicht (B) repräsentiert den Verbesserungsfaktor (vorliegend 7.5) für eine Detektion mit einem Photomultiplier.

In Bezug auf die Zeichnung wird vorliegend als eine besonders bevorzugte Ausführungsform die Verwendung einer optischen Anordnung 10 und des Verfahrens gemäß dem Konzept der Erfindung zur CARS (coherent anti-Stokes Ramans scattering) - Spektroskopie beschrieben. Es wird demonstriert, dass es möglich ist, auch schwache CARS-Signale von bisher nicht erreichbaren Molekülen unterhalb einer Milli-Molar-Konzentration im Bereich der Schrotrausch-Grenze (Shot noise limited) im Rahmen eines besonders kompakten optischen Aufbaus zu detektieren, wobei die Phasenkohärenz des CARS-Prozesses zur heterodynen Detektion genutzt wird. Der Aufbau der optischen Anordnung 10 und das Verfahren erweisen sich hinsichtlich ihrer Kompaktheit bzw. ihres Rauschverhaltens als Überlegen gegenüber bisher bekannten Konzepten des Standes der Technik.

Obwohl vorliegend die Eingangswellenlängen, welche das CARS-Signal erzeugen - nämlich eine fundamentale Laserfrequenz und eine Idler-Frequenz - keine direkte Phasenbeziehung haben, so ist dennoch die Ausgangswellenlänge des CARS-Signals phasengelockt zu einer Wellenlänge des OPO-Signals ist, welche simultan mit dem Idler-Signal im optischen parametrischen Oszillator des in Fig. 1 gezeigten Aufbaus erzeugt wird, um ein angemessenes sogenanntes lokales Oszillatorfeld zur heterodynen Detektion des CARS-Signals zu erzeugen. Eine Analyse der Rauscheigenschaften des Detektionsschemas eröffnet eine Nische, welche es erlaubt das CARS-Signal an der Schrotrausch-Grenze zu detektieren. Zusätzliche Messungen unter Abschwächung des CARS-Signals zeigen, dass die Detektion des CARS-Signalpegels deutlich unter dem Detektorrauschpegel (Dunkelpegel) und den aktuellen Detektorgrenzen liegt. Der Aufbau der optischen Anordnung 10 ist inhärent frei von einem zeitlichen Jitter der korrelierten Pulse und nutzt jede der im Rahmen des Aufbaus erzeugte Wellenlänge zur Erzeugung und Detektion des CARS-Signals.

Fig. 1 zeigt dazu die phasenerhaltende nicht-lineare Kette des Aufbaus der optischen Anordnung 10 als eine besonders bevorzugte Ausführung einer Vorrichtung zum Erzeugen und Detektieren des nicht-linearen optischen Signals 17 in Form des CARS-Signals. Dieser weist eine erste optische Generatoreinheit 1 in Form eines Oszillators zur Erzeugung von Pulsen einer ersten Frequenz in einem ersten Strahl 3 auf. Der erste optische Oszillator ist dazu in Form eines passiv modengelockten Nd:YAG-Lasers zur Erzeugung von Pulsen einer Pulsbreite von etwa 15 ps bei einer Repetitionsrate nahe von 80 MHz gebildet. Die zentrale Wellenlänge der Pulse liegt bei 1064 nm und diese eine fundamentale Frequenz vorgebende Wellenlänge wird teilweise in einem vorliegend extern im Strahl 3 vorgesehenen Frequenzverdoppler 18 zur Erzeugung von Pulsen 5 von 12ps Breite bei 532 nm genutzt. Der frequenzverdoppelte Teil des Strahls 3 wird als phasenkohärenter Pumpstrahl 5 zum Pumpen einer zweiten optischen Generatoreinheit 7 in Form eines optischen parametrischen Oszillators dem Eingang des optischen parametrischen Oszillators über entsprechende Spiegel in einem Pumpzweig 6 der geeignet ausgelegten Strahlführung zugeführt. Die Phasenkohärenz ergibt sich durch Addition der Phasen der zwei die zweite harmonische Frequenz (532 nm) bildenden Photonen. Der somit bei 532 nm synchron gepumpte optische parametrische Oszillator generiert in einer in Fig. 1 vorliegend schematisch dargestellten Ausführung Pulspaare mit einer Pulsbreite von etwa 6,5 ps einerseits bei einer Signalwellenlänge eines Signalstrahls 9 und andererseits einer Idler-Wellenlänge eines Idler-Strahls 8. Die Phasen der korrelierten Pulse des Signalstrahls 9 und des Idler-Strahls 8 sind für sich frei, aber die Addition ist gelockt an die Phase des Pumpstrahls 3. Diese Freiheit der Phase des Signalstrahls 9 gewährleistet, dass das im Resonator unterstützte Signal keine Phasenbegrenzung hat und somit einen glatten Betrieb des optisch parametrischen Oszillators (OPO) ermöglicht. Die Wellenlänge des Idler-Strahls 8 wird in den den ersten Strahl 3 führenden Zweig 11 der Strahlführung mit den 1064 nm des ersten Strahls 3 zur Erzeugung eines Anregungsfeldes 2 und eines CARS-Signals bei einer Frequenz von

$$\Omega_{CARS} = 2\Omega_{pump} - \Omega_{Stokes}$$

kombiniert, wobei die 1064 nm als Pumpfrequenz und die Frequenz des Idlers-Strahls 8 als Stokeswellenlänge agiert.

Die CARS- (oder anti-Stokes) Wellenlängen entspricht wie aus Ansicht B der Fig. 1 ersichtlich der Wellenlänge des Signalstrahls 9 des OPOs. Des weiteren bestimmt sich die Phase des resonanten CARS-Signals praktisch in der gleichen Weise wie die Phase des Signalstrahls 9 des OPOs (2 x $Phase_{1064}$ - $Phase_{Idler}$ + $Phase_{Chi(3)}$), wobei der letzte Term eine konstante Phase der nicht-linearen Antwort Chi(3) bei einer bestimmten Wellenlänge ist. Der Phasenerhalt bedeutet, dass die Signalwellelänge mit dem CARS-Signal in einer vorhersagbaren Weise im Interferenzstrahl 4 des zweiten Zweigs 12 der Strahlführung interferieren kann und somit zur interferometischen Verstärkung genutzt werden kann.

Die Interferenz des lokalen Oszillatorfeldes (LO, OPO-Signalwellenlänge 9), mit dem CARS-Signal (CARS) führt zu einer gesamten Intensität auf den Detektor von:

$$I_{Detector} = LO + CARS + 2 \times \sqrt{(LO \cdot CARS)},$$
$$wobei\ HCARS = 2 \times \sqrt{(LO \cdot CARS)},$$

wobei LO und CARS Intensitäten darstellen. HCARS bezieht sich auf die heterodyne CARS-Leistung. Diese Interferenz-Term skaliert mit der Wurzel der Leistung des LO-Feldes und die interferometrische Verstärkung kann als HCARS/ CARS = 2 x $\sqrt{(LO/CARS)}$ definiert werden. Das Rauschen des optischen Signals bestimmt sich durch das Schrot-Rauschen im CARS-Signal selbst und das Schrot-Rauschen, welches durch das lokale Oszillator-Signal, d.h. das OPO-Signal 9 eingeführt wird. Das eingeführte Schrot-Rauschen ist der dominierende Term der zwei (weil LO sehr viel größer als CARS ist) und skaliert mit der Wurzel von LO, ähnlich wie der Interferenz-Term und die interferometrische Verstärkung.. Die interferometrische Verstärkung kann also genutzt werden, um das Signal über das Detektorrauschen zu heben, ohne das ursprüngliche Signal-zu-Rausch-Verhältnis zu degradieren solange das lokale Oszillator-Feld LO der für das Schrot-Rauschen dominante Term ist. Um ein 1/f-Rauschen zu vermeiden, wird die Detektion in einen spektral weniger rauschanfälligen Bereich gebracht, indem die CARS-Wellenlänge um 40kHZ in Bezug auf die OPO-Wellenlänge verschoben wird.

Der Detektor 19 kann entweder ein Photo-Multiplier (PMT), eine Photo-Diode (PD) oder eine Avalanche-Photo-Diode (APD) sein. APDs werden im allgemeinen genutzt in einem Zählmodus für Photonen und erzeugen für jedes detektierte Photon einen elektronischen Puls. Der Photo-Multiplier und die Photo-Diode werden mit einem Transimpedanz-Verstärker verbunden, welcher bei 0 Ohm durch einen Operationsverstärker (OPAMP) abgeschlossen ist, wie dies der Einschub in Fig. 2 zeigt. Die Photo-Diode PD kann als eine Stromquelle angesehen werden, mit einem Strom proportional zur detektierten Intensität (typischerweise 0,6 A/W). Die dominierenden Rauschquellen im Detektor sind das Johnson-Rauschen im Widerstand ($\sqrt{4kTR \cdot BW}$, wobei BW die Detektionsbandbreite in Hz darstellt), das Verstärkerstromrauschen (Dunkelstrom unabhängig von R) und das Schrot-Rauschen der Anzahl von Elektronen, welche durch das detektierte Signal generiert werden. Die detektierte Spannung skaliert linear mit dem Transimpedanz-Widerstand (R). Durch Erhöhen des Widerstands bis zu einem Punkt, wo dieser die anderen Rauschquellen dominiert, skaliert das Signal-zu-Rausch-Verhältnis des Verstärkers mit $\sqrt{R}$ und für einen ausreichend großen Output wird das Rauschen dominiert durch das Schrot-Rauschen des detektierten Signals. Das Maximum von R ist begrenzt durch die benötigte Bandbreite des Verstärkers, welche mit 1/R skaliert. Eine typische Konfiguration erreicht eine 1MHz-Bandbreite für 1 M$\Omega$ Transimpedanz. Der Photo-Multiplier PMT ist ähnlich wie die Photo-Diode PD ausgelegt, außer dass der Dunkelstrom geringer ist wenn er verglichen wird mit einem Widerstand ähnlicher Verstärkung. Bei einer Verstärkung von $10^7$ hat ein Photo-Multiplier typischerweise 100 Photonen x BW Dunkelzählratenrauschen (10 Kilo/s Dunkelzähler). Avalanche-Photo-Dioden APD haben die geringste Dunkelzählrate (ungefähr 10 Photonen x BW).

Wenn die Menge an Leistung der lokalen Oszillator-Stärke LO, 9 am Detektor erhöht wird, zeigt das Rauschen (Signal ohne jedes CARS-Signal am Detektor) zuerst den Dunkelzählrauschpegel (flach), gefolgt von einem Abschnitt, welcher das Schrot-Rauschen des lokalen Oszillatorfeldes zeigt. Dieses zeigt sich als eine Steigung ½ in einer doppelt-logarythmischen Darstellung zur LO-Leistung (parallel zur Linie A in Fig.2). An einem bestimmten Punkt wird das Rauschen dominiert durch die Amplitudenfluktuation des lokalen Oszillatorfeldes LO, 9. Die Amplitudenfluktuation sind korreliert zur Leistung des lokalen Oszillators LO, 9 und skalieren deshalb mit einer Steigung von 1 (parallel zur Linie B). Da dieser Abschnitt des Rauschens schneller skaliert als das heterodyne Signal 4 (letzteres skaliert parallel zur Linie A) nimmt das Signal-zu-Rauschverhältnis ab. Das Fenster, in welchem CARS-Signale nur begrenzt durch das Schrot-Rauschen detektiert werden können, steigt dort, wo das Rauschen der Linie A folgt. Das Fenster wird begrenzend abgeschlossen durch das von den Amplitudenfluktuationen verursachte Rauschen.

Der Verstärkungsdynamik im OPO bestimmt die Amplitudenfluktuationen, insbesondere den Sättigungspegel. Wenn der OPO nicht stark gesättigt ist, können die Amplitudenfluktuationen bei einem (lokalen Oszillator-) Pegel auftreten, wo das Rauschen noch nicht durch das Schrot-Rauschen dominiert ist und effektiv das Fenster zur schrotrauschenbegrenzten Detektion schließen.

Der Pegel des detektierten Schrot-Rauschens im Vergleich zum realen optischen Schrot-Rauschen skaliert invers mit der Wurzel der Quanteneffizienz (QE), was den Teil die Linie aufwärts schiebt. Die Position der Amplitudenfluktuationen bleibt unberührt und skaliert direkt mit der Anzahl der Photonen. Dies scheint das Detektionsfenster zu verbessern, indem höhere Pegel eines lokalen Oszillatorfeldes LO, 9 erlaubt sind, aber in der Realität verschlechtert sich die Detektionssensivität, wie Fig. 4 zeigt. Photo-Multiplier tendieren dazu, eine geringe Quanteneffizienz (QE 10 % bis 0,1 %) für Wellenlängen nahe des nahen Infrarots (NIR) zu haben. Photo-Dioden und Avalanche-Photo-Dioden haben eine sehr viel bessere Quanteneffizienz (QE typischerweise bis zu 85 %). Für die Detektion von sehr geringen Signalen erweisen sich deshalb Avalanche-Photo-Dioden als die vergleichsweise beste Wahl. Für praktische Anwendungen können Photo-Dioden favorisiert werden, weil sie groß, preiswert und robust sind. Avalanche-Photo-Dioden sind vergleichsweise teuer und zerbrechlich und haben einen vergleichsweise reduzierten dynamischen Bereich. Solange ein Fenster existiert, in dem eine schrotrauschenlimitierte Detektion möglich ist, ist die Wahl des Detektors vergleichsweise unkritisch. Dies ist jedoch anders, wenn das Fenster durch einen oben beschriebenen Prozess vergleichsweise klein wird.

Vorliegend ist die Wellenlänge des OPO-Idlerstrahls 8 auf 1578 nm abgestimmt, so dass die Differenzfrequenz zwischen den 1064 nm des ersten Strahls 3 und den 1578 nm des Idler-Strahls 9 mit einer C-H Streck-Vibrationsanregung bei 3060cm$^{-1}$ in Toluenen übereinstimmt. Das OPO-Signal 9 (ebenso das CARS-Signal) liegt bei einer Wellenlänge von 802.7 nm. Die Toluene-Probe (Material 16) wird zwischen Deckgläsern präpariert und ist etwa 15 $\mu$m dick. Einige zehn Milliwatt bei 1064 nm und 1578 nm werden unter Nutzung eines 0.60NA Luftobjektivs 13.1 fokussiert. Das CARS-Signal wird mit einem 0,65NA Objektiv 13.2 gesammelt. Die Filter 13.3 entfernen die 1578nm und 1064 nm Anteile der Anregungsfelder 3,8.

Um die CARS-Frequenz zu verschieben, wird ein Akusto-optischer Modulator 14 AOM benutzt, welcher in dem 1064 nm ersten Strahls 3 - eher als im Signalstrahl 9 platziert wird, weil dessen Wellenlänge sich nicht ändert im Zuge einer weiteren Abstimmung des OPOs auf eine bestimmte vibronische Frequenz. Der AOM wird bei 80 MHz betrieben und schiebt effektiv die 1064 nm Pulse bei einer Repetitionsrate von 80.02 MHz um 20kHz. Diese Verschiebung setzt sich um in eine Verschiebung von 40kHz bei der CARS-Wellenlänge aufgrund der Beteiligung von zwei Photonen der 1064 nm im ersten Strahl 3 im Anregungsprozess. Die CARS-Wellenlänge (CARS) wird an einem Detektor 19 (PD, APD, PMT) mit dem OPO-Signal (LO), 9 bei der oben genannten Wellenlänge zum Interferenzsignal 4 kombiniert und die detektierte Intensität wird an einen Lock-In-Verstärker 15 weitergegeben, welcher zur Detektion bei 40kHZ eingestellt ist. Eine Integrationszeit von 100 ms unter Nutzung eines Kantenfilters zweiter Ordnung (BW = 1,58 Hz) wird verwendet.

Fig. 2 zeigt die detektierten Rauschpegel für eine Photo-Diode mit drei verschiedenen Transimpedanzen und einem Photo-Multiplier als Funktion einer Leistung des lokalen Oszillator-Feldes LO. Die Rauschpegel werden zurückgerechnet auf eine rauschäquivalente Eingangsleistung (bei 803 nm, 1,58Hz Bandbreite) als Vergleich. Die Schrot-Rauschlinie für den Photo-Multiplier liegt deutlich über der für Photo-Dioden aufgrund der geringen Quanteneffizienz (QE = 0,3 %). Der Photo-Multiplier zeigt ein großes Fenster für eine schrotrauschenbegrenzte Detektion. Die Photo-Dioden zeigen ein Fenster, nur wenn diese bei 1MΩ oder 10MΩ eingestellt sind. Bei geringerer Einstellung führt das benötigte lokale Oszillator-Feld LO Rauschpegel durch Amplitudenfluktuation dominiertes Rauschen ein. Oberhalb von 10MΩ ist es dem OPAMP nicht mehr möglich, den 40kHz-Oszillationen im Rahmen der Verstärkung zu folgen.

Die Abhängigkeit der Amplitudenfluktuationspegel von der Sättigung des OPOs wird in Fig. 3 demonstriert, wo sowohl die Leistung des Signals 9 am Ausgang des OPOs als auch das detektierte Rauschen bei der heterodynen Frequenz für einen festen Pegel des lokalen Oszillatorfeldes LO am Detektor (2μmW) gezeigt ist. Pumpen des OPOs bei 5 W und 532 nm anstatt 2 W und 532 nm erhöht die Sättigung. Das Abnehmen in den Amplitudenfluktuationen ermöglicht eine schrotrauschenbegrenzte Detektion bei geringerer Transimpedanz (und entsprechend höherer Frequenz) bis das Eingangs-Spannungsrauschen das Johnson-Rauschen dominiert (unterhalb 50kΩ).

In einer Abwandlung hat sich insbesondere die Verwendung eines LBO-OPO's als vorteilhaft erwiesen, der einen höheren Sättigungspegel hat. Moderne Pumpquellen für den OPO können mit geeignet kurzen Pulsen eine Grenzleistung am OPO-Ausgang bei etwa 400mW oder mehr ermöglichen. Eine Sättigung kann dann bei dem drei- oder vierfachen der Grenzleistung erreicht werden, z.B. bei 1 Watt bis 1,5 Watt Leitung am OPO-Ausgang.

Fig. 4 zeigt ein gemessenes nicht-lineares Signal 17 als ein heterodynes CARS-Signal bei einem festen lokalen Oszillator-Pegel (50 nW für die Photo-Diode und 2 nW für den Photo-Multiplier), wobei das CARS-Signal (CARS) durch Einführen von Abschwächungsfiltern 13.3 im entsprechenden Teil des Strahls variiert wird. Zum Vergleich wird auch die direkte Detektion des CARS-Signals bei entferntem lokalen Oszillatorfeld (LO, 9) gezeigt, wobei stattdessen der CARS-Strahl des nicht-linearen Signals mit einem mechanischen Chopper gechoppt wird. Das gechoppte Signal zeigt die erwartete direkte Abhängigkeit am CARS-Pegel mit der Steigung 1. Das heterodyne Signal 4 hängt nur von der Wurzel des CARS-Signals ab und nimmt somit mit einer Steigung von 1/2 ab. Mit zunehmender Abschwächung des CARS-Pegels verschwindet das direkt detektierte Signal im Detektorrauschen, während das heterodyne Detektieren sich bei geringeren Pegeln fortsetzt bevor es im lokalen Oszillatorrauschen verschwindet. Für eine Detektion mit einer bei 10MΩ abgestimmte Photo-Diode liegt das minimal detektierbare CARS-Signal unter Verwendung einer heterodynen Detektion um einen Faktor 3500 unterhalb des Signals, welches direkt detektiert werden kann. Der Photo-Multiplier hat einen sehr geringen Rauschpegel und eine hohe Verstärkung, so dass diese Verbesserung durch die heterodyne Detektion nicht so eindrucksvoll ist, aber dennoch bei einem Faktor von 7.5 liegt. Allerdings ist üblicherweise eine Quanteneffizienz (QE) des PhotoMultipliers vergleichsweise gering, so dass das Minimum eines detektierbaren Signals deutlich über dem liegt, was durch heterodyne Detektion unter Benutzung einer Photo-Diode detektiert werden kann. Es gibt Photo-Multiplier mit einer Quanteneffizienz von bis 15 %. Für diese Photo-Multiplier würde das heterodyne Detektionsminimum das Limit der Photo-Diode erreichen. Aufgrund verbleibender Abgleichungsfehler ist ein solches detektierte heterodyne Signal um einen Faktor 10 unterhalb des theoretischen Limits.

Die rauschfreie heterodyne Verstärkung wird erreicht für einen Pegel des lokalen Oszillators (LO), welcher derart gewählt ist, dass das lokale Oszillatorrauschen nur wenig oberhalb des Detektordunkelrauschpegels liegt. In der Praxis heißt das, dass vergleichsweise geringe Pegel (nW) des lokalen Oszillators LO erforderlich sind, um das Signal über das Detektorrauschen zu heben, im Vergleich zu Lichtpegel, welche das CARS-Sginal treiben (mW). Der lokale Oszillator (LO) kann also in einer Abwandlung in kollinearer Konfiguration mit den anderen Strahlen 3, 8 durch eine Materialprobe gesendet werden, ohne den CARS-Prozess zu stören, was vorteilhaft Laufzeitunterschiede bei einer scannenden Mikroskopie vermeidet.

Für eine Avalanche-Diode ist der Vorteil einer heterodynen Detektion geringer, da die Rauschpegel geringer sind und die Quanteneffizienz höher ist. Jedoch ist es wichtig festzustellen, dass das Stromdetektionslimit nicht durch das Detektorrauschen allein verursacht ist. Für eine sehr geringe Anzahl von resonanten Molekülen im Fokusvolumen kann der nicht-resonante Hintergrund das resonante Signal übersteigen ähnlich wie eine Auto-Fluoreszenz die Detektion einer Fluoreszenz-Probe begrenzt. In dem eingangs genannten Artikel von Potma et.al. ist gezeigt, dass - da der resonante Signalanteil in der Phase verschoben ist im Vergleich zum nicht-resonanten Signalanteil - das interferometrische Signal verwendet werden kann, um die resonanten Anteile des CARS-Signals von den nicht-resonanten Anteilen zu trennen und diesen Hintergrund zu unterdrücken. Um auch eine Rauschverstärkung des nicht-resonanten Signals zu vermeiden, können die Polarisationsrichtungen der einfallenden Strahlen 3, 8 und des lokalen Oszillators 9 aneinander angepasst werden, um nur den resonanten Teil des CARS-Signals im Rahmen der optischen Interferenz zu verstärken. Der Gesamtverlust am Signal wird kompensiert durch die interferometrische Verstärkung und das durch den nicht-resonanten Anteil generierte Rauschen addiert sich nicht zum Rauschen des resonanten Anteils. Eine solche Kombination einer heterodynen Interferenz und eines Polarisation-CARS Detektionsschemas (HIPCARS) erweist sich auch als vorteilhaft.

Zusammenfassend wurde die Möglichkeit einer rauschfreien Verstärkung für ein nicht-lineares Signal (CARS) in Form eines CARS-Signals beispielhaft demonstriert, was den OPO zur Realisierung eines optischen Aufbaus 10 in einer Multistrahlkonfiguration zur heterodynen Detektion vorteilhafter macht, dies bevorzugt im Vergleich zu den eingangs genannten Lösungen - welche beispielsweise eine nicht-lineare Glasfaser oder einen Referenz-CARS-Aufbau mit einer d-DMSO-Probe oder einem anderen Material mit hohem Chi(3) nutzen und bei denen die Rauschpegel völlig unspezifiziert sind. Außerdem ergibt sich mit dem vorliegend dargestellten beispielhaften optischen Aufbau 10 eine inhärent zeitliche jitterfreie Betriebsmöglichkeit, so dass die Möglichkeit besteht, einen Wellenlängscan ohne Synchronisationsfehler durchzuführen und die Möglichkeit besteht, resonante und nicht-resonante Beiträge zu trennen. Dieses wird die Anwendbarkeit einer Vorrichtung der erläuterten Art im Rahmen der CARS-Mikroskopie signifikant verbessern.

Die Erfindung geht aus von einem Verfahren und einer optischen Anordnung 10 zum Erzeugen eines nicht-linearen optischen Signals (CARS) an einem durch ein Anregungsfeld 2 angeregten Material, wobei mit dem Anregungsfeld 2 kohärente Felder optischer Pulse unterschiedlicher Frequenz in dem Material zeitlich und örtlich überlappt werden, wobei erste Pulse einer ersten Frequenz in einem ersten Strahl 3 einer ersten optischen Generatoreinheit 1 und zweite Pulse einer zweiten Frequenz in einem zweiten Strahl 8 einer von der ersten optischen Generatoreinheit 1 synchron gepumpten, zweiten optischen Generatoreinheit 7 erzeugt werden. Gemäß dem Konzept der Erfindung sieht das Verfahren vor, dass mit den Pulsen der ersten Frequenz als fundamentale Frequenz (1064 nm) Pulse einer höheren harmonischen Frequenz (532 nm) derselben erzeugt und die zweite optische Generatoreinheit 7 mit den Pulsen auf der höheren harmonischen Frequenz gepumpt wird und die zweite optische Generatoreinheit 7 als optische parametrische Generatoreinheit Pulse einer Idler-Frequenz (1578 nm) in einem Idler-Strahl zur Bereitstellung der zweiten Pulse der zweiten Frequenz in dem zweiten Strahl 8 erzeugt, wobei die zweite Frequenz (1578 nm) kleiner als die erste Frequenz (1064 nm) ist und die zweite optische Generatoreinheit 7 Pulse einer Signalfrequenz (802.7 nm) in einem Signalstrahl 9 erzeugt, wobei die zweite Frequenz (1578 nm) kleiner als die Signalfrequenz (802.7 nm) ist.

## Patentansprüche

1. Verfahren zum Erzeugen eines nicht-linearen optischen Signals (17) an einem durch ein Anregungsfeld (2) angeregten Material (16), wobei mit dem Anregungsfeld (2) kohärente Felder erster und zweiter optischer Pulse unterschiedlicher Frequenz in dem Material (16) zeitlich und örtlich überlappt werden, wobei die ersten Pulse einer ersten Frequenz in einem ersten Strahl (3) einer ersten optischen Generatoreinheit (1) und
die zweiten Pulse einer zweiten Frequenz in einem zweiten Strahl (8) einer von der ersten optischen Generatoreinheit (1) synchron gepumpten, zweiten optischen Generatoreinheit (7) erzeugt werden, wobei mit den ersten Pulsen der ersten Frequenz als fundamentale Frequenz Pulse einer höheren harmonischen Frequenz derselben erzeugt (SHG) und die zweite optische Generatoreinheit mit den Pulsen (5) der höheren harmonischen Frequenz gepumpt wird,
**dadurch gekennzeichnet, dass**
die zweite optische Generatoreinheit (7) als optische parametrische Generatoreinheit Pulse einer Idler-Frequenz in einem Idler-Strahl zur Bereitstellung der zweiten Pulse der zweiten Frequenz in dem zweiten Strahl (8) erzeugt, wobei die zweite Frequenz kleiner als die erste Frequenz ist und
die zweite optische Generatoreinheit (7) Pulse einer Signal-Frequenz in einem Signal-Strahl (9) erzeugt, wobei die zweite Frequenz kleiner als die Signal-Frequenz ist, und wobei das nicht-lineare optische Signal (17) zur Detektion heterodyn mit dem Signal-Strahl (9) interferierend überlappt wird.

2. Verfahren nach Anspruch 1,
**dadurch gekennzeichnet, dass**
die Summe aus Idler-Frequenz und Signal-Frequenz der höheren harmonischen Frequenz, insbesondere der Frequenz einer zweiten Harmonischen, entspricht.

3. Verfahren nach Anspruch 1 oder 2,
**dadurch gekennzeichnet, dass**
das nicht-lineare optische Signal (17), insbesondere in Form eines CARS-Signals, bei einer Frequenz gebildet wird, welche der doppelten der ersten Frequenz minus der Idler-Frequenz entspricht.

**4.** Verfahren nach einem der Ansprüche 1 bis 3,
**dadurch gekennzeichnet, dass**
eine optische Phasenlage zwischen einem Puls des Signal-Strahls (9) und dem nicht-linearen optischen Signal (17) definiert ist, insbesondere eine Phasenlage zwischen einem Puls des ersten Strahls (3) und einem Puls des zweiten Strahls (8) undefiniert ist.

**5.** Verfahren nach einem der Ansprüche 1 bis 4,
**dadurch gekennzeichnet, dass**
eine Frequenz des nicht-linearen optischen Signals (17) relativ zur Signal-Frequenz des Signal-Strahls (9) verändert und das nichtlineare optische Signal (17) über ein zeitabhängiges interferierendes Signal (4) detektiert wird.

**6.** Verfahren nach einem der Ansprüche 1 bis 5,
**dadurch gekennzeichnet, dass**
eine Frequenz und/oder Phase des nicht-linearen optischen Signals (17) relativ zur Signal-Frequenz und/oder Phase verändert wird, indem die erste Frequenz und/oder Phase geändert wird, insbesondere durch ein frequenzmodulierendes und/oder phasenmodulierendes Mittel, z. B. eine akusto-optische Modulation (AOM).

**7.** Verfahren nach einem der Ansprüche 1 bis 6,
**dadurch gekennzeichnet, dass**
der erste Strahl (3) und der zweite Strahl (8) kollinear überlappt werden und/oder der Signal-Strahl (9) oder ein Teil desselben mit dem nicht-linearen optischen Signal (17), insbesondere in einem Detektor (19), kollinear überlappt wird und/oder
der Signal-Strahl (9) oder ein Teil desselben mit dem ersten Strahl (3) und zweiten Strahl (8) kollinear, insbesondere im Material (16), überlappt wird.

**8.** Verfahren nach einem der Ansprüche 1 bis 7,
**dadurch gekennzeichnet, dass**
die Intensität des Signal-Strahls (9) derart gering gewählt wird, dass eine Stärke des nicht-linearen optischen Signals (17) unbeeinflusst vom Signal-Strahl (9) ist.

**9.** Verfahren nach einem der Ansprüche 1 bis 7,
**dadurch gekennzeichnet, dass**
die Intensität des Signal-Strahls (9) derart hoch gewählt wird, dass eine Stärke des nicht-linearen optischen Signals (17) mit der Intensität des Signal-Strahls (9) ansteigt.

**10.** Verfahren nach einem der Ansprüche 1 bis 9,
**dadurch gekennzeichnet, dass**
die Felder des ersten Strahls (3) und zweiten Strahls (8) kopolarisiert oder nichtkopolarisiert, z.B. kreuzpolarisiert, werden oder so gewählt werden, dass das Anregungsfeld (2) zirkular oder elliptisch polarisiert ist.

**11.** Optische Anordnung (10) zum Erzeugen eines nicht-linearen optischen Signals an einem durch ein Anregungsfeld (2) angeregten Material (16), wobei mit dem Anregungsfeld (2) kohärente erste und zweite Felder optischer Pulse unterschiedlicher Frequenz in dem Material (16) zeitlich und örtlich überlappbar sind, insbesondere mittels einem Verfahren nach Anspruch 1, aufweisend:

- eine erste optische Generatoreinheit (1) zum Erzeugen der ersten Pulse einer ersten Frequenz in einem ersten Strahl (3),
- ein Mittel (18) zum Erzeugen von Pulsen (5) einer höheren harmonischen Frequenz mit den ersten Pulsen der ersten Frequenz als fundamentale Frequenz,
- eine von der ersten optischen Generatoreinheit (1) synchron mit den Pulsen (5) der höheren harmonischen Frequenz pumpbare, zweite optische Generatoreinheit (7) in Form einer optischen parametrischen Generatoreinheit zum Erzeugen von Pulsen einer Idler-Frequenz in einem Idler-Strahl als zweite Pulse in dem zweiten Strahl (8) auf einer zweiten Frequenz und zum Erzeugen von Pulsen einer Signal-Frequenz in einem Signal-Strahl (9), wobei die zweite Frequenz kleiner als die erste Frequenz und kleiner als die Signal-Frequenz ist,
- Strahlführungsmittel (11) zum zeitlichen und örtlichen Überlappen eines ersten Pulses des ersten Strahls und eines zweiten Pulses des Idler-Strahls,
- weitere Strahlführungsmittel (12) ausgelegt zum Detektieren des nicht-linearen optischen Signals durch heterodynes, interferierendes Überlappen mit dem Signal-Strahl (9).

**12.** Optische Anordnung nach Anspruch 11, **gekennzeichnet durch** ein Mittel (14) zur Veränderung der Frequenz des nicht-linearen optischen Signals relativ zur Signal-Frequenz des Signal-Strahls und einen Detektor (19) und weitere Strahlführungsmittel (12) zum heterodynen, interferierenden Überlappen des nicht-linearen optischen Signals (17) mit dem Signal-Strahl (9) in dem Detektor (PD, APD, PMT).

**13.** Optische Anordnung nach Anspruch 11 oder 12, **dadurch gekennzeichnet, dass** die erste optische Generatoreinheit (1) in Form eines Lasers, insbesondere einem Nd:YAG- oder einem Ti:Saphir-Laser, gebildet ist, der insbesondere zur Erzeugung von Pikosekunden-Pulsen und/oder Femtosekunden-Pulsen ausgelegt ist.

**14.** Optische Anordnung nach einem der Ansprüche 11 bis 13, **dadurch gekennzeichnet, dass** die zweite optische Generatoreinheit (7) in Form eines optischen parametrischen Oszillators, insbesondere eines KTP-OPO's oder LBO-OPO's, gebildet ist oder die zweite optische Generatoreinheit (7) in Form eines optischen parametrischen Generators oder eines optischen parametrischen Verstärkers gebildet ist, der insbesondere zur Erzeugung von Pikosenkunden-Pulsen und/oder Femtosekunden-Pulsen ausgelegt ist.

**15.** Optische Anordnung nach einem der Ansprüche 11 bis 14, **dadurch gekennzeichnet, dass** das Mittel (18) zum Erzeugen von Pulsen einer höheren harmonischen Frequenz in Form eines Frequenzverdopplers, insbesondere mit einem oder mehreren nicht-linearen Kristallen gebildet ist.

**16.** Optische Anordnung nach einem der Ansprüche 11 bis 15, **dadurch gekennzeichnet, dass** das Mittel zum Erzeugen einer höheren harmonischen Frequenz zwischen der ersten optischen Generatoreinheit (1) und der zweiten optischen Generatoreinheit (7) angeordnet ist und/oder das Mittel zum Erzeugen einer höheren harmonischen Frequenz in einem Resonator der ersten optischen Generatoreinheit gebildet ist.

**17.** Optische Anordnung nach einem der Ansprüche 11 bis 16, **dadurch gekennzeichnet, dass** ein Mittel (14) zur Veränderung der Frequenz und/oder Phase des nicht-linearen optischen Signals relativ zur Frequenz und/oder Phase des Signal-Strahls als ein Mittel zur Veränderung der fundamentalen Frequenz und/oder Phase im ersten Strahl (3), und/oder der Idler-Frequenz und/oder Phase im Idler-Strahl (8), und/oder der Signal-Frequenz und/oder Phase im Signal-Strahl (9), angeordnet ist, insbesondere in Form eines akusto-optischen Modulators (AOM) gebildet ist.

**18.** Verwendung eines Verfahrens nach einem der Ansprüche 1 bis 10 und/oder einer optischen Anordnung (19) nach einem der Ansprüche 11 bis 17 zur Mikroskopie und/oder Spektroskopie, insbesondere CARS-Mikroskopie und/oder CARS-Spektroskopie.

**Claims**

**1.** Method of generating a non-linear optical signal (17) on a material (16) excited by an excitation field (2), fields of first and second optical pulses of differing frequency and coherent with the excitation field (2) being temporally and spatially overlapped in the material (16), the first pulses of a first frequency being generated in a first beam (3) of a first optical generator unit (1), and the second pulses of a second frequency being generated in a second beam (8) of a second optical generator unit (7) synchronously pumped by the first optical generator unit (1), the first pulses of the first frequency, as a fundamental frequency, generating pulses of a higher harmonic frequency thereof (SHG) and the second optical generator unit being pumped with the pulses (5) of the higher harmonic frequency, **characterised in that** the second optical generator unit (7), as an optical parametric generator unit, generates pulses of an idler frequency in an idler beam for providing the second pulses of the second frequency in the second beam (8), the second frequency being smaller than the first frequency, and the second optical generator unit (7) generates pulses of a signal frequency in a signal beam (9), the second frequency being smaller than the signal frequency, and for detection the non-linear optical signal (17) being overlapped in a heterodyne manner with the signal beam (9), so as to interfere therewith.

2. Method according to claim 1,
   **characterised in that**
   the sum of the idler frequency and the signal frequency corresponds to the higher harmonic frequency, in particular the frequency of a second harmonic.

3. Method according to either claim 1 or claim 2,
   **characterised in that**
   the non-linear optical signal (17), in particular in the form of a CARS signal, is formed at a frequency which corresponds to double the first frequency minus the idler frequency.

4. Method according to any one of claims 1 to 3,
   **characterised in that**
   an optical phase position is defined between a pulse of the signal beam (9) and the non-linear optical signal (17), in particular a phase position is undefined between a pulse of the first beam (3) and a pulse of the second beam (8).

5. Method according to any one of claims 1 to 4,
   **characterised in that**
   a frequency of the non-linear optical signal (17) is altered relative to the signal frequency of the signal beam (9) and the non-linear optical signal (17) is detected by way of a time-dependent interfering signal (4).

6. Method according to any one of claims 1 to 5,
   **characterised in that**
   a frequency and/or phase of the non-linear optical signal (17) is altered relative to the signal frequency and/or phase **in that** the first frequency and/or phase is altered, in particular by a frequency-modulating and/or phase-modulating means, for example an acousto-optical modulator (AOM).

7. Method according to any one of claims 1 to 6,
   **characterised in that**
   the first beam (3) and the second beam (8) are collinearly overlapped and/or the signal beam (9) or a part thereof is collinearly overlapped with the non-linear optical signal (17), in particular in a detector (19), and/or
   the signal beam (9) or a part thereof is collinearly overlapped with the first beam (3) and the second beam (8), in particular in the material (16).

8. Method according to any one of claims 1 to 7,
   **characterised in that**
   the intensity of the signal beam (9) is selected to be low such that a strength of the non-linear optical signal (17) is uninfluenced by the signal beam (9).

9. Method according to any one of claims 1 to 7,
   **characterised in that**
   the intensity of the signal beam (9) is selected to be high such that a strength of the non-linear optical signal (17) rises with the intensity of the signal beam (9).

10. Method according to any one of claims 1 to 9,
    **characterised in that**
    the fields of the first beam (3) and the second beam (8) are copolarised or non-copolarised, for example cross-polarised, or are selected such that the excitation field (2) is circularly or elliptically polarised.

11. Optical arrangement (10) for generating a non-linear optical signal (17) on a material (16) excited by an excitation field (2), first and second fields of optical pulses of differing frequency and coherent with the excitation field (2) being temporally and spatially overlapped in the material (16), in particular by means of a method according to claim 1, comprising:

    - a first optical generator unit (1) for generating the first pulses of a first frequency in a first beam (3),
    - a means (18) for generating pulses (5) of a higher harmonic frequency with the first pulses of the first frequency as a fundamental frequency,

- a second optical generator unit (7) which can be pumped by the first optical generator unit (1) synchronously with the pulses (5) of the higher harmonic frequency and is in the form of an optical parametric generator unit for generating pulses of an idler frequency in an idler beam as second pulses in the second beam (8) to a second frequency and for generating pulses of a signal frequency in a signal beam (9), the second frequency being smaller than the first frequency and smaller than the signal frequency,

- beam guide means (11) for temporal and spatial overlapping of a first pulse of the first beam and a second pulse of the idler beam,

- further beam guide means (12) designed to detect the non-linear optical signal (17) by heterodyne interfering overlapping with the signal beam (9).

12. Optical arrangement according to claim 11,
**characterised by**
a means (14) for altering the frequency of the non-linear optical signal relative to the signal frequency of the signal beam and a detector (19) and further beam guide means (12) for heterodyne interfering overlapping of the non-linear optical signal (17) with the signal beam (9) in the detector (PD, APD, PMT).

13. Optical arrangement according to either claim 11 or claim 12,
**characterised in that**
the first optical generator unit (1) is in the form of a laser, in particular an Nd:YAG or a Ti:sapphire laser, which is designed in particular to generate picosecond pulses and/or femtosecond pulses.

14. Optical arrangement according to any one of claims 11 to 13,
**characterised in that**
the second optical generator unit (7) is in the form of an optical parametric oscillator, in particular a KTP-OPO or LBO-OPO or
the second optical generator unit (7) is in the form of an optical parametric generator or an optical parametric amplifier, which is designed in particular to generate picosecond pulses and/or femtosecond pulses.

15. Optical arrangement according to any one of claims 11 to 14,
**characterised in that**
the means (18) for generating pulses of a higher harmonic frequency is in the form of a frequency doubler, in particular with one or more non-linear crystals.

16. Optical arrangement according to any one of claims 11 to 15,
**characterised in that**
the means for generating a higher harmonic frequency is arranged between the first optical generator unit (1) and the second optical generator unit (7) and/or
the means for generating a higher harmonic frequency is formed in a resonator of the first optical generator unit.

17. Optical arrangement according to any one of claims 11 to 16,
**characterised in that**
a means (14) for altering the frequency and/or phase of the non-linear optical signal relative to the frequency and/or phase of the signal beam is arranged as a means for altering the fundamental frequency and/or phase in the first beam (3) and/or the idler frequency and/or phase in the idler beam (8) and/or the signal frequency and/or phase in the signal beam (9), and in particular is in the form of an acousto-optical modulator (AOM).

18. Use of a method according to any one of claims 1 to 10 and/or an optical arrangement (19) according to any one of claims 11 to 17 for microscopy and/or spectroscopy, in particular CARS microscopy and/or CARS spectroscopy.

## Revendications

1. Procédé pour la production d'un signal (17) optique non-linéaire sur un matériau (16) excité par un champ d'excitation (2), dans lequel des champs cohérents de premières et de secondes impulsions optiques de fréquence différente dans le matériau (16) sont superposés dans le temps et localement avec le champ d'excitation (2), les premières impulsions d'une première fréquence étant générées dans un premier faisceau (3) d'un premier générateur (1) optique et
les secondes impulsions d'une seconde fréquence étant générées dans un second faisceau (8) d'un second générateur (7) optique, pompé de façon synchrone par le premier générateur (1) optique, des impulsions d'une fréquence harmonique supérieure de ce générateur étant générées avec les premières impulsions de la première fréquence en tant que fréquence fondamentale (SHG) et le second générateur optique étant pompé avec les impulsions (5) de la fréquence harmonique supérieure,

**caractérisé en ce que**

le second générateur (7) optique génère en tant que générateur paramétrique optique des impulsions d'une fréquence idler dans un faisceau idler pour la fourniture des secondes impulsions de la seconde fréquence dans le second faisceau (8), la seconde fréquence étant inférieure à la première fréquence et

le second générateur (7) optique génère des impulsions d'une fréquence signal dans un faisceau signal (9), la seconde fréquence étant inférieure à la fréquence signal, et le signal (17) optique non-linéaire étant superposé pour la détection hétérodyne en interférence avec le faisceau signal (9).

2. Procédé selon la revendication 1,
   **caractérisé en ce que**
   la somme résultant de la fréquence idler et de la fréquence signal correspond à la fréquence harmonique supérieure, en particulier la fréquence d'une seconde harmonique.

3. Procédé selon la revendication 1 ou 2,
   **caractérisé en ce que**
   le signal (17) optique non-linéaire, en particulier sous la forme d'un signal CARS, est formé à une fréquence qui correspond au double de la première fréquence moins la fréquence idler.

4. Procédé selon l'une quelconque des revendications 1 à 3,
   **caractérisé en ce que**
   une relation de phase optique est définie entre une impulsion du faisceau signal (9) et le signal (17) optique non-linéaire, en particulier une relation de phase est indéfinie entre une impulsion du premier faisceau (3) et une impulsion du second faisceau (8).

5. Procédé selon l'une quelconque des revendications 1 à 4,
   **caractérisé en ce**
   **qu'**une fréquence du signal (17) optique non-linéaire varie par rapport à la fréquence signal du faisceau signal (9) et le signal (17) optique non-linéaire est détecté par un signal (4) interférent et dépendant du temps.

6. Procédé selon l'une quelconque des revendications 1 à 5,
   **caractérisé en ce que**
   une fréquence et/ou une phase de signal (17) optique non-linéaire est modifiée par rapport à la fréquence signal et/ou la phase du fait que la première fréquence et/ou phase est modifiée, en particulier par un moyen modulant la fréquence et/ou modulant la phase, par exemple une modulation acousto-optique (AOM).

7. Procédé selon l'une quelconque des revendications 1 à 6,
   **caractérisé en ce que**
   le premier faisceau (3) et le second faisceau (8) sont superposés de façon colinéaire et/ou le faisceau signal (9) ou une partie de ce faisceau est superposé(e) de façon colinéaire avec le signal (17) optique non-linéaire, en particulier dans un détecteur (19),
   et/ou
   le faisceau signal (9) ou une partie de ce faisceau est superposé(e) avec le premier faisceau (3) et le second faisceau (8) de façon colinéaire, en particulier dans le matériau (16).

8. Procédé selon l'une quelconque des revendications 1 à 7,
   **caractérisé en ce que**
   l'intensité du faisceau signal (9) est choisie tellement basse qu'une puissance du signal (17) optique non-linéaire n'est pas influencée par l'intensité du faisceau signal (9).

9. Procédé selon l'une quelconque des revendications 1 à 7,
   **caractérisé en ce que**
   l'intensité du faisceau signal (9) est choisie tellement élevée qu'une puissance du signal (17) optique non-linéaire augmente avec l'intensité du faisceau signal (9).

**10.** Procédé selon l'une quelconque des revendications 1 à 9,
**caractérisé en ce que**
les champs du premier faisceau (3) et du second faisceau (8) sont copolarisés ou non copolarisés, par exemple polarisés de façon croisée, ou sont choisis de façon que le champ d'excitation (2) soit polarisé de façon circulaire ou elliptique.

**11.** Agencement (10) optique pour la production d'un signal optique non-linéaire sur un matériau (16) excité par un champ d'excitation (2), des premiers et seconds champs cohérents d'impulsions optiques de fréquence différente dans le matériau (16) pouvant être superposés dans le temps et localement avec le champ d'excitation (2), en particulier au moyen d'un procédé selon la revendication 1, présentant :

- un premier générateur (1) optique pour la production des premières impulsions d'une première fréquence dans un premier faisceau (3),
- un moyen (18) pour la production d'impulsions (5) d'une fréquence harmonique supérieure avec les premières impulsions de la première fréquence comme fréquence fondamentale,
- un second générateur (7) optique, pouvant être pompé par le premier générateur (1) optique de façon synchrone avec les impulsions (5) de la fréquence harmonique supérieure, sous la forme d'un générateur paramétrique optique pour la production d'impulsions d'une fréquence idler dans un faisceau idler comme secondes impulsions dans le second faisceau (8) sur une seconde fréquence et pour la production d'impulsions d'une fréquence signal dans un faisceau signal (9), la seconde fréquence étant inférieure à la première fréquence et inférieure à la fréquence signal,
- des moyens de guidage de faisceau (11) pour la superposition dans le temps et locale d'une première impulsion du premier faisceau et d'une seconde impulsion du faisceau idler,
- d'autres moyens de guidage de faisceau (12) conçus pour la détection du signal optique non-linéaire par superposition hétérodyne et interférente avec le faisceau signal (9).

**12.** Agencement optique selon la revendication 11,
**caractérisé par**
un moyen (14) pour modifier la fréquence du signal optique non-linéaire par rapport à la fréquence signal du faisceau signal et un détecteur (19) et d'autres moyens de guidage de faisceau (12) pour la superposition hétérodyne et interférente du signal (17) optique non-linéaire avec le faisceau signal (9) dans le détecteur (PD, APD, PMT).

**13.** Agencement optique selon la revendication 11 ou 12,
**caractérisé en ce que**
le premier générateur (1) optique est formé sous la forme d'un laser, en particulier d'un laser Nd:YAG ou d'un laser Ti:Saphir, qui est conçu en particulier pour la production d'impulsions de l'ordre de la picoseconde et/ou d'impulsions de l'ordre de la femtoseconde.

**14.** Agencement optique selon l'une quelconque des revendications 11 à 13,
**caractérisé en ce que**
le second générateur (7) optique est formé sous la forme d'un oscillateur paramétrique optique, en particulier d'un KTP-OPO ou d'un LBO-OPO ou
le second générateur (7) optique est formé sous la forme d'un générateur paramétrique optique ou d'un amplificateur paramétrique optique, qui est conçu en particulier pour la production d'impulsions de l'ordre de la picoseconde et/ou d'impulsions de l'ordre de la femtoseconde.

**15.** Agencement optique selon l'une quelconque des revendications 11 à 14,
**caractérisé en ce que**
le moyen (18) pour la production d'impulsions d'une fréquence harmonique supérieure est formé sous la forme d'un doubleur de fréquence, en particulier avec un ou plusieurs cristaux non linéaires.

**16.** Agencement optique selon l'une quelconque des revendications 11 à 15,
**caractérisé en ce que**
le moyen pour la production d'une fréquence harmonique supérieure est disposé entre le premier générateur (1) optique et le second générateur (7) optique et/ou
le moyen pour la production d'une fréquence harmonique supérieure est formé dans un résonateur du premier générateur optique.

**17.** Agencement optique selon l'une quelconque des revendications 11 à 16,
**caractérisé en ce que**
un moyen (14) pour modifier la fréquence et/ou la phase du signal optique non-linéaire par rapport à la fréquence et/ou la phase du faisceau signal est disposé comme moyen pour modifier la fréquence fondamentale et/ou la phase dans le premier faisceau (3), et/ou la fréquence idler et/ou la phase dans le faisceau idler (8) et/ou la fréquence signal et/ou la phase dans le faisceau signal (9), en particulier est formé sous la forme d'un modulateur acousto-optique (AOM).

**18.** Utilisation d'un procédé selon l'une quelconque des revendications 1 à 10 et/ou d'un agencement optique (19) selon l'une quelconque des revendications 11 à 17, pour la microscopie et/ou la spectroscopie, en particulier la microscopie CARS et/ou la spectroscopie CARS.

EP 2 153 210 B1

(A)

1
Modelocked Nd

18
SHG

3

3, 5
1064nm

5
532nm

10

14
AOM
3

3

7

11

Idler
8

3, 8

OPO

Signal

LO, 9

LO, Signal, 9

6

12

13.1  2  16  13.2    13.3

Material     Filters

17, CARS

4

(B)

1064    1064    Signal    Idler

1064

SHG + OPO

CARS

1064    Idler'    1064    CARS

Detektor
19        PD, APD, PMT

Lock-in -
Verstärker        15

FIG. 1

FIG. 2

FIG. 3

EP 2 153 210 B1

FIG. 4

# EP 2 153 210 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **F. Ganikhanov.** *Optics Letters,* 2006, vol. 31 (12), 1872-1874
- **E.O. Potma.** Heterodyne coherent anti-Stokes Raman scattering (CARS) imaging. *Opt.Lett.,* 14. Januar 2006, vol. 31 (2), 241-243
- **E.R. Andresen.** Picosecond anti-Stokes generation in a photonic-crystal fiber for interferometric CARS microscopy. *Opt. Expr,* 2006, vol. 14 (16), 7246-7251
- CARS imaging with a new 532 nm synchroneously pumped picosecond OPO. **von Büttner.** Proc. of SPIE Conf. Multiphoton Microscopy in the Biomedical Sciences VII. 21. Januar 2007, vol. 6442, S64420C-164420C-8